# EUROPEAN PATENT APPLICATION

(11) **EP 3 824 874 A1**
(43) Date of publication of application: **26.05.2021**
(21) Application number: 19838666.6
(22) Date of filing: 21.06.2019
(51) Int. Cl.: A61K 8/81, A61K 8/31, A61K 8/37, A61K 8/41, A61K 8/89, A61Q 17/00

(54) **COMPOSITION FOR PREVENTING ADHESION OF AIR-BORNE FINE PARTICLES**

(30) Priority: 20.07.2018 JP 2018136830
(71) Applicant: Shiseido Company, Ltd., Chuo-ku Tokyo 104-0061 (JP)
(72) Inventor: MIYAZAWA, Kazuyuki, Tokyo 104-0061 (JP); ARINO, Shoko, Tokyo 104-0061 (JP); SHIRAKAMI, Hirohito, Tokyo 104-0061 (JP); HORIMOTO, Maki, Tokyo 104-0061 (JP)
(74) Representative: Ter Meer Steinmeister & Partner
(86) International application number: PCT/JP2019/024652
(87) International publication number: WO 2020/017232

(57) **Abstract**

The present invention relates to a composition for preventing adhesion of air-borne fine particles, wherein the composition contains:
(A) a 2-(meth)acryloyloxyalkyl phosphorylcholine / (meth)acrylic acid alkyl ester copolymer;
(B) a di-chain cationic surfactant represented by formula (I) below, 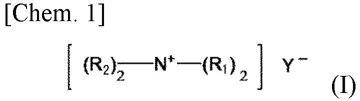 where R₁ denotes, each independently, an alkyl group having 12 to 22 carbon atoms and 0 to 3 double bonds, R₂ denotes, each independently, an alkyl group having 1 to 3 carbon atoms and lacking a double bond, and Y denotes a halogen atom, a methosulfate or a methophosphate; and
(C) one or more oils selected from among hydrocarbon oils, ester oils and silicone oils.

## Description

### TECHNICAL FIELD

The present invention relates to a composition for preventing the adhesion of air-borne fine particles. More specifically, the present invention relates to a composition for preventing the adhesion of air-borne fine particles that suppresses the adhesion, to clothes, skin, hair and the like, of harmful matter that is suspended in air, such as pollen, viruses and PM2.5.

### BACKGROUND ART

Fine particles (air-borne fine particles) that are suspended in air, including allergens such as pollen, viruses such as influenza viruses, and particulate matter such as PM2.5 and aeolian dust, cause various health problems by contacting or entering the human body. For example, there is the risk that allergens such as pollen and house dust will cause various allergy symptoms, and viruses such as influenza will cause infectious diseases. Additionally, there are reports that particulate matter having a particle size of 2.5 µm or smaller (so-called PM2.5) that is discharged by the burning of matter induces respiratory system diseases, circulatory system diseases and the like.

As means for protecting the body from air-borne fine particles, it is common to physically shut out or remove air-borne fine particles by using a mask, an air purifier or the like. However, in recent years, there have been proposals to make use of the fact that most air-borne fine particles are charged by applying a composition containing an antistatic agent to clothes, hair or the like, thereby actively removing static electricity.

For example, JP 2004-068174 A (Patent Document 1) describes a fiber irritation suppressant containing a phosphorylcholine analog group-containing polymer or a polyalkylene glycol derivative polymer, exhibiting a molecular weight and a surface tension that are in specified ranges. The document indicates that, by processing fibers with a fiber irritation suppressant containing such a polymer, allergic reactions to allergenic matter from pollen, mites and the like can be reduced.

Meanwhile, JP 2006-2147 A (Patent Document 2) describes that a polymer containing, as constituent units, monomer units having specific amphoteric ion groups and/or monomer units having specific anion groups has the functions and effects of preventing the adhesion of pollen to hair, clothes and the like. This polymer can also be sprayed or applied not only to fibers such as those in clothes and bed linens, but also to hair or the like. As a specific example of the polymer, a 2-(meth)acryloyloxyethyl phosphorylcholine / (meth)acrylic acid butyl ester copolymer is mentioned. This polymer is mixed with water, ethanol or the like, and prepared as a lotion or a spray.

The polymer described in Patent Document 2 can safely be applied directly to hair or the like in addition to clothes. Thus, the application thereof to cosmetics or the like for use on skin might be contemplated. However, the present inventors have observed that, when a formulation containing the polymer described in Patent Document 2 is blended with an oil, the adhesion prevention effects against air-borne fine particles such as pollen are reduced.

Oils have moisturizing effects in themselves, and can stably dissolve oil-soluble agents such as oil-soluble ultraviolet absorbers. For this reason, if an oil could be blended, then various functions such as moisturization and ultraviolet protection could be imparted.

Thus, in the composition containing the polymer described in Patent Document 2, a means for allowing a sufficient amount of an oil to be blended without reducing the air-borne fine particle adhesion prevention effects is sought.

### RELATED ART

### PATENT DOCUMENTS

Patent Document 1: JP 2004-68174 A
Patent Document 2: JP 2006-2147 A

### SUMMARY OF THE INVENTION

### PROBLEM TO BE SOLVED BY THE INVENTION

An objective of the present invention is to provide a composition containing, as an antistatic agent, the polymer in Patent Document 2, namely, a 2-(meth)acryloyloxyalkyl phosphorylcholine / (meth)acrylic acid alkyl ester copolymer, wherein the air-borne fine particle adhesion prevention effects of the composition are not reduced even when a sufficient amount of an oil is blended therein.

### MEANS FOR SOLVING THE PROBLEM

As a result of performing diligent research towards solving the above-mentioned problem, the present inventors discovered that, by blending a prescribed cationic surfactant with a prescribed oil, a sufficient amount of an oil can be blended into a composition containing a 2-(meth)acryloyloxyalkyl phosphorylcholine / (meth)acrylic acid alkyl ester copolymer, without reducing the air-borne fine particle adhesion prevention effects.

In other words, the present invention is mainly directed to a composition for preventing adhesion of air-borne fine particles, wherein the composition contains:
(A) a 2-(meth)acryloyloxyalkyl phosphorylcholine / (meth)acrylic acid alkyl ester copolymer;
(B) a di-chain cationic surfactant represented by formula (I) below, 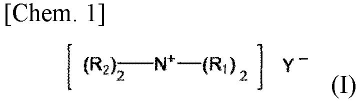 where R₁ denotes, each independently, an alkyl group having 12 to 22 carbon atoms and 0 to 3 double bonds, R₂ denotes, each independently, an alkyl group having 1 to 3 carbon atoms and lacking a double bond, and Y denotes a halogen atom, a methosulfate or a methophosphate; and
(C) one or more oils selected from among hydrocarbon oils, ester oils and silicone oils.

### EFFECTS OF THE INVENTION

In the present invention, a di-chain cationic surfactant and a specific oil are blended in a composition containing a 2-(meth)acryloyloxyalkyl phosphorylcholine / (meth)acrylic acid alkyl ester copolymer as an antistatic agent, thereby allowing a sufficient amount of the oil to be blended without reducing the air-borne fine particle adhesion prevention effects.

As a result thereof, in addition to being able to add the moisturizing effects of the oil itself to the composition, oil-soluble agents can also be stably blended, thereby allowing various functions to be imparted to the composition.

### MODES FOR CARRYING OUT THE INVENTION

The composition for preventing adhesion of air-borne fine particles according to the present invention essentially contains (A) a 2-(meth)acryloyloxyalkyl phosphorylcholine / (meth)acrylic acid alkyl ester copolymer, (B) a di-chain cationic surfactant, and (C) a prescribed oil. Hereinafter, the ingredients constituting the composition of the present invention will be explained in detail.

### <(A) 2-(Meth)acryloyloxyalkyl phosphorylcholine / (meth)acrylic acid alkyl ester copolymer>

The (A) 2-(meth)acryloyloxyalkyl phosphorylcholine / (meth)acrylic acid alkyl ester copolymer (hereinafter sometimes referred to simply as "component (A)") blended in the composition according to the present invention corresponds to one form of the polymer that is described as having a pollen adhesion prevention effect in the above-mentioned Patent Document 2.

Component (A) is a copolymer of monomer units comprising 2-(meth)acryloyloxyalkyl phosphorylcholine and monomer units comprising (meth)acrylic acid alkyl ester, where the "alkyl" is a lower alkyl having 1 to 6 carbon atoms. Additionally, the weight-average molecular weight of component (A) is preferably 500,000 to 800,000, more preferably 600,000 to 700,000.

A favorable example of component (A) is a 2-(meth)acryloyloxyethyl phosphorylcholine / (meth)acrylic acid butyl ester copolymer. A commercially available product may also be used. For example, "Lipidure-PMB" (manufactured by NOF Corporation) or the like may be used.

The blended amount of component (A) should be 0.01% to 5.0% by mass, preferably 0.05% to 3.0% by mass relative to the total mass of the composition. If the amount is less than 0.01% by mass, then there are cases in which sufficient air-borne fine particle adhesion prevention effects cannot be obtained. On the other hand, if the amount exceeds 5.0% by mass, then time is required for evaporation and there are cases in which there can be stickiness to the touch when dry. As component (A), it is possible to use one type alone or to use a combination of two or more types of the above-mentioned 2-(meth)acryloyloxyalkyl phosphorylcholine / (meth)acrylic acid alkyl ester copolymers.

### <(B) Di-chain cationic surfactant>

As the (B) di-chain cationic surfactant (hereinafter sometimes referred to simply as "component (B)") blended in the composition according to the present invention, the di-chain cationic surfactant represented by formula (I) below can be favorably used.

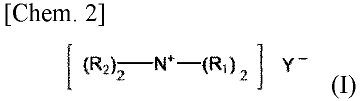

In the above formula (I), R₁ denotes, each independently, an alkyl group having 12 to 22 carbon atoms and 0 to 3 double bonds, R₂ denotes, each independently, an alkyl group having 1 to 3 carbon atoms and lacking a double bond, and Y denotes a halogen atom, a methosulfate or a methophosphate.

Although the di-chain cationic surfactant represented by formula (I) above is not limited, examples include dibehenyl dimethyl ammonium chloride, distearyl dimethyl ammonium chloride (commercially available as "Cation DSV" (manufactured by Sanyo Chemical Industries Co., Ltd.) etc.), dicetyl dimethyl ammonium chloride, dicetostearyl dimethyl ammonium chloride, distearyl dimethyl ammonium methosulfate, dibehenyl dimethyl ammonium methosulfate, dicetyl dimethyl ammonium methosulfate, dicetostearyl dimethyl ammonium methosulfate and the like. Among the above, distearyl dimethyl ammonium chloride is preferably used.

The blended amount of component (B) should be 0.001% to 0.1% by mass, preferably 0.005% to 0.05% by mass relative to the total mass of the composition. If the amount is less than 0.001% by mass, then there are cases in which the reduction in air-borne fine particle adhesion prevention effects cannot be sufficiently suppressed when an oil is blended. On the other hand, if the amount exceeds 0.1% by mass, then there are cases in which stickiness occurs, conversely causing air-borne fine particles to be more easily adhered. As component (B), it is possible to use one type alone or to use a combination of two or more types of the above-mentioned di-chain cationic surfactants.

### <(C) Oil>

The (C) oil (hereinafter sometimes referred to simply as "component (C)") blended in the composition according to the present invention is one or more oils selected from among hydrocarbon oils, ester oils and silicone oils.

Examples of hydrocarbon oils include isododecane, isohexadecane, isoparaffin, liquid paraffin (mineral oil), ozokerite, squalane, paraffin, ceresin, squalene, vaseline, microcrystalline wax and the like.

Examples of ester oils include cetyl ethylhexanoate, triethylhexanoin, isopropyl myristate, octyldodecyl myristate, isopropyl palmitate, butyl stearate, hexyl laurate, myristyl myristate, decyl oleate, isocetyl stearate, cholesteryl 12-hydroxystearate, neopentyl glycol dicaprate, PPG-3 dipivalate, diisostearyl malate, di-2-ethylhexyl succinate, triisostearin, trimethylolpropane triisostearate, oleyl oleate, diisobutyl adipate, hexyldecyl palmitate, diisopropyl sebacate, pentaerythrityl tetraethylhexanoate, jojoba oil, macadamia nut oil and the like.

Examples of silicone oils include linear polysiloxanes (for example, dimethyl polysiloxane, methylphenyl polysiloxane, diphenyl polysiloxane and the like), cyclic polysiloxanes (for example, octamethyl cyclotetrasiloxane, decamethyl cyclopentasiloxane, dodecamethyl cyclohexasiloxane and the like) and the like.

Among the above, the (C) oil is preferably an oil that, in particular, has an IOB of 0.55 or lower, more preferably an IOB of 0.52 or lower.

The IOB refers to the ratio (Inorganic Organic Balance) of the inorganic value (IV) to the organic value (OV), i.e., "Inorganic value (IV)/Organic value (OV)", on the organic conception diagram. The organic conception diagram is obtained by supposing that methane (CH₄) is the basis for all organic compounds and that all other compounds are derivatives of methane, setting fixed numerical values for the number of carbons, the substituent groups, modifications, rings and the like thereof, adding up the score to determine organic values and inorganic values, then plotting the organic values on the the X axis and the inorganic values on the Y axis (see Yukigainenzu-Kiso to Oyo-[The Organic Conception Diagram -Fundamentals and Applications-] (by Yoshio KODA, Sankyo Publishing, 1984)).

Examples of oils that are particularly favorable as component (C) include dimethicone (IOB = 0.19), diphenylsiloxyphenyl trimethicone (IOB = 0.16), mineral oil (IOB = 0.00), vegetable squalane (IOB = 0.00), cetyl ethylhexanoate (IOB = 0.13), triethylhexanoin (IOB = 0.35), pentaerythrityl tetraethylhexanoate (IOB = 0.35), hydrogenated polyisobutene (IOB = 0.00), neopentyl glycol dicaprate (IOB = 0.25), triisostearin (IOB = 0.16), diisostearyl malate (IOB = 0.28), di-2-ethylhexyl succinate (IOB = 0.32), PPG-3 dipivalate (IOB = 0.52) and the like.

Among the above, dimethicone (IOB = 0.19), mineral oil (IOB = 0.00), cetyl ethylhexanoate (IOB = 0.13) and PPG-3 dipivalate (IOB = 0.52) are particularly preferable.

The blended amount of component (C) should be 0.01% to 1% by mass, preferably 0.05% to 0.5% by mass relative to the total mass of the composition. If the amount is less than 0.01% by mass, then there are cases in which the effects of blending an oil cannot be obtained, such as not being able to stably dissolve various types of oil-soluble agents. On the other hand, if the amount exceeds 1% by mass, then there are cases in which stickiness occurs, conversely causing air-borne fine particles to be more easily adhered. As component (C), it is possible to use one type alone or to use a combination of two or more types of the above-mentioned oils.

### <Other blendable components>

The composition for preventing adhesion of air-borne fine particles according to the present invention may further contain, as needed, within a range not compromising the effects of the present invention, various types of solvents, surfactants, oil-soluble agents, ultraviolet absorbers, higher alcohols, thickeners, essential oils, moisturizers, antioxidants, metal sequestrants, pH adjusters, fragrances, preservatives, propellants and the like.

Examples of solvents include water, lower alcohols such as ethanol and propanol, polyhydric alcohols such as glycerin, propylene glycol and 1,3-butylene glycol, volatile oils such as low-boiling-point linear silicone oils, cyclic silicone oils and low-boiling-point isoparaffin-based hydrocarbon oils, and the like.

As the surfactant, for example, it is possible to use one or more types selected from among non-ionic surfactants that are conventionally used in oil-in-water emulsion cosmetics, and polyoxyethylene hardened castor oils are particularly preferable. Specific examples of polyoxyethylene hardened castor oils include PEG-10 hydrogenated castor oil, PEG-20 hydrogenated castor oil, PEG-25 hydrogenated castor oil, PEG-30 hydrogenated castor oil, PEG-40 hydrogenated castor oil, PEG-50 hydrogenated castor oil, PEG-60 hydrogenated castor oil, PEG-80 hydrogenated castor oil, PEG-100 hydrogenated castor oil and the like.

Examples of oil-soluble agents include oil-soluble agent components such as whiteners, moisturizers, anti-inflammatory agents, antimicrobial agents, hormonal agents, vitamins, various amnio acids and derivatives thereof, enzymes, antioxidants, hair nurturing agents, ultraviolet absorbers and the like. Specific examples include vitamin A (retinol) and derivatives thereof (for example, retinol acetate, retinol palmitate and the like), vitamin B2 derivatives (for example, riboflavin acetic acid esters and the like), vitamin B6 derivatives (for example, pyridoxine dicaprate, pyridoxine dipalmitate, pyridoxine dilaurate and the like), vitamin D (calciferol) and derivatives thereof (for example, ergocalciferol, cholecalciferol and the like), vitamin E (tocopherol) and derivatives thereof (for example, vitamin E acetate (tocopherol acetate) and the like), essential fatty acids (for example, linolic acid, linoleic acid, arachidonic acid, eicosapentaenoic acid, docosahexaenoic acid and vegetable extracts (for example, *Platycladus orientalis* seeds and the like) containing the above, and the like), ubiquinone (coenzyme Q10) and derivatives thereof, vitamin K (for example, phylloquinone, menaquinone, menadione and the like), resorcin derivatives (for example, 4-alkylresorcinol derivatives and/or salts thereof and the like), glycyrrhetinic acid and derivatives thereof (for example, stearyl glycyrrhetinate and the like), oil-soluble vitamin C derivatives (for example, vitamin C dipalmitate (ascorbyl dipalmitate), ascorbyl stearate and the like), steroid compounds (for example, female hormones, male hormones and the like), benzyl nicotinate (a hair nurturing agent component), trichlorocarbanilide (a microbicidal component), trichlorohydroxydiphenyl ether (a preservative component), γ-oryzanol (an antioxidant component), dibutylhydroxytoluene (an antioxidant component), and octyl methoxycinnamate, 4-(1,1-dimethylethyl)-4'-methoxybenzoyl methane and octocrylene as ultraviolet absorbers, and the like. However, there is no limitation to these examples. One or more of the oil-soluble agents may be used.

### <Composition for preventing adhesion of air-borne fine particles>

The composition for preventing adhesion of air-borne fine particles according to the present invention may be provided in the form of an oil-in-water emulsion cosmetic. The specific format may be an arbitrary format such as a spray, a lotion or the like, which may be produced by using conventional methods appropriate for the respective formats.

The effects of the composition for preventing adhesion of air-borne fine particles according to the present invention are achieved by depositing the composition by spraying or by application to a desired target surface. For example, the composition may be deposited on a portion of the body, including the face or hair, or may be deposited on clothes or a portable article such as a handkerchief, a mask or the like.

The composition for preventing adhesion of air-borne fine particles according to the present invention suppresses the adhesion of air-borne fine particles by actively removing static electricity from the areas to which it is applied. The air-borne fine particles that can be prevented from adhesion include harmful matter that is suspended in air in general, and are not particularly limited. Examples include allergens such as pollen and house dust, viruses such as influenza viruses, and particulate matter such as PM2.5, aeolian dust and asbestos.

### EXAMPLES

Hereinafter, the present invention will be explained in further detail by providing examples. However, the present invention is not limited to these examples in any way. Where not otherwise noted, the blended amounts are indicated in percentage by mass relative to the system in which the component is blended.

### <Experimental Examples 1 to 4>

Lotions having the compositions shown in Table 1 below were prepared in accordance with conventional methods, and the pollen adhesion prevention effects thereof were investigated in accordance with the evaluation methods indicated below. The evaluation results are also shown in Table 1.

### <Pollen adhesion prevention effects>

Lycopodium powder (the spores of *Lycopodium clavatum*), which is a pseudopollen, was used as air-borne fine particles to evaluate how well the adhesion of lycopodium powder to a vinyl chloride sheet could be prevented, as indicated below.

### (i) Preparation of test pieces

Vinyl chloride sheets (1.5 cm × 4.5 cm) were prepared and clipped to clips suspended by string. The vinyl chloride sheets were immersed in sample solutions of the respective experimental examples, then dried for 1 hour at room temperature.

### (ii) Pollen adhesion test

20 mg of lycopodium powder was put in a 100 ml screw tube. The string to which a test piece was connected was fixed to the lid of the screw tube, and the lid was closed, taking care to keep the test piece from coming into contact with the inner walls of the screw tube. The lid of the screw tube was held and the screw tube was placed upright at the center of a vortex mixer (model "SI-0286" (Scientific Industries, Inc.); rotation speed 1000 rpm) and shaken for 10 seconds. The amount of pollen that adhered was observed by eye, compared with a control (Experimental Example 1 in Table 1 and Experimental Example 5 in Table 2) containing only water and ethanol, and assessed under the criteria below.

### (iii) Assessment criteria

A: Much less pollen adhesion than control
B: Slightly less pollen adhesion than control
C: Same amount of pollen adhesion as control
D: More pollen adhesion than control

In the present invention, in the cases of an assessment of A or B, the reduction in the pollen adhesion prevention effect was considered to have been sufficiently suppressed, thus a "pass" was recorded. Otherwise, a "fail" was recorded.

**[Table 1]**

| | Exp. Ex. 1 | Exp. Ex. 2 | Exp. Ex. 3 | Exp. Ex. 4 |
|---|---|---|---|---|
| Water | 70 | 69.88 | 69.28 | 69.27 |
| Ethanol | 30 | 30 | 30 | 30 |
| 2-(meth)acryloyloxyethyl phosphorylcholine / (meth)acrylic acid butyl ester copolymer | - | 0.1 | 0.1 | 0.1 |
| Phenoxyethanol | - | 0.02 | 0.02 | 0.02 |
| Cetyl ethylhexanoate (IOB = 0.13) | - | - | 0.1 | 0.1 |
| Distearyl dimethyl ammonium chloride | - | - | - | 0.01 |
| PEG-60 hydrogenated castor oil | - | - | 0.5 | 0.5 |
| Total | 100 | 100 | 100 | 100 |
| Pollen adhesion prevention effect | C | A | D | B |

As indicated in Table 1, compared to the case in which a 2-(meth)acryloyloxyethyl phosphorylcholine / (meth)acrylic acid butyl ester copolymer was not blended (Experimental Example 1), the pollen adhesion prevention effects were largely improved when said copolymer was blended (Experimental Example 2). Although the pollen adhesion prevention effects were significantly degraded when an oil was blended (Experimental Example 3), sufficient pollen adhesion prevention effects were able to be maintained by blending distearyl dimethyl ammonium chloride (Experimental Example 4).

### <Experimental Examples 5 to 13>

Lotions having the compositions shown in Table 2 below were prepared in accordance with conventional methods, and the pollen adhesion prevention effects were investigated in accordance with the evaluation method indicated above. As the amount of ethanol was reduced, the drying time when preparing the test pieces was set to 3 hours. The evaluation results are also indicated in Table 2.

**[Table 2]**

| | Exp. Ex. 5 | Exp. Ex. 6 | Exp. Ex. 7 | Exp. Ex. 8 | Exp. Ex. 9 | Exp. Ex. 10 | Exp. Ex. 11 | Exp. Ex. 12 | Exp. Ex. 13 |
|---|---|---|---|---|---|---|---|---|---|
| Water | 85 | 84.8 8 | 84.2 8 | 84.2 7 | 84.3 9 | 84.1 6 | 84.2 7 | 84.2 7 | 84.2 6 |
| Ethanol | 15 | 15 | 15 | 15 | 15 | 15 | 15 | 15 | 15 |
| 2-(meth)acryloyloxyeth yl phosphorylcholine / (meth)acrylic acid butyl ester copolymer | - | 0.1 | 0.1 | 0.1 | - | 0.2 | 0.1 | 0.1 | 0.1 |
| Phenoxyethanol | - | 0.02 | 0.02 | 0.02 | - | 0.04 | 0.02 | 0.02 | 0.02 |
| Cetyl ethylhexanoate (IOB = 0.13) | - | - | 0.1 | 0.1 | 0.1 | 0.1 | 0.1 | 0.1 | 0.1 |
| Dicocoylethyl hydroxyethylmonium methosulfate | - | - | - | - | - | - | 0.01 | - | - |
| Distearyl dimethyl ammonium chloride | - | - | - | 0.01 | 0.01 | - | - | - | - |
| PEG-10 dimethicone | - | - | - | - | - | - | - | 0.01 | - |
| Benzalkonium chloride | - | - | - | - | - | - | - | - | 0.02 |
| PEG-60 hydrogenated castor oil | - | - | 0.5 | 0.5 | 0.5 | 0.5 | 0.5 | 0.5 | 0.5 |
| Total | 100 | 100 | 100 | 100 | 100 | 100 | 100 | 100 | 100 |
| Pollen adhesion prevention effect | C | A | D | B | D | D | D | C | C |

As indicated in Table 2, although sufficient pollen adhesion prevention effects were able to be maintained by blending distearyl dimethyl ammonium chloride (Experimental Example 8), the reduction in the pollen adhesion prevention effects due to the blending of the oil could not be suppressed in the case in which another surfactant that is commonly used in cosmetic products was used (Experimental Examples 11 to 13). Additionally, although it is known that distearyl dimethyl ammonium chloride itself has antistatic effects, pollen adhesion prevention effects were not obtained when a 2-(meth)acryloyloxyethyl phosphorylcholine / (meth)acrylic acid butyl ester copolymer was not included (Experimental Example 9).

### <Experimental Examples 14 to 19>

In order to investigate the effects of cationic surfactants other than the (B) di-chain cationic surfactant, lotions having the compositions shown in Table 3 below were prepared in accordance with conventional methods, (i) test pieces were prepared and (ii) pollen adhesion tests were performed in the same manner as the above-mentioned pollen adhesion prevention effect evaluation method, the change in the pollen adhesion amount compared with a control (Experimental Example 14) not containing a cationic surfactant was observed by eye, and assessments were made in accordance with the criteria below.

### (iii) Assessment criteria

A: Pollen adhesion amount clearly reduced
B: Almost no change in pollen adhesion amount could be observed
C: Pollen adhesion amount conversely increased.

**[Table 3]**

| | Exp. Ex. 14 | Exp. Ex. 15 | Exp. Ex. 16 | Exp. Ex. 17 | Exp. Ex. 18 | Exp. Ex. 19 |
|---|---|---|---|---|---|---|
| Water | 1.88 | 1.88 | 1.88 | 1.88 | 1.88 | 1.88 |
| 2-(meth)acryloyloxyethyl phosphorylcholine / (meth)acrylic acid butyl ester copolymer | 0.1 | 0.1 | 0.1 | 0.1 | 0.1 | 0.1 |
| Phenoxy ethanol | 0.02 | 0.02 | 0.02 | 0.02 | 0.02 | 0.02 |
| Cetyl ethylhexanoate (IOB = 0.13) | 0.1 | 0.1 | 0.1 | 0.1 | 0.1 | 0.1 |
| Ethanol | 97.9 | 97.85 | 97.85 | 97.85 | 97.85 | 97.85 |
| Distearyl dimethyl ammonium chloride | - | 0.05 | - | - | - | - |
| Benzalkonium chloride | - | - | 0.05 | - | - | - |
| Cetyl trimethyl ammonium bromide | - | - | - | 0.05 | | - |
| Stearyl trimethyl ammonium chloride | - | - | - | - | 0.05 | - |
| Benzyl trimethyl ammonium bromide | - | - | - | - | - | 0.05 |
| Total | 100 | 100 | 100 | 100 | 100 | 100 |
| Change in pollen adhesion amount | - | A | C | B | C | C |

As indicated in Table 3, the pollen adhesion amount was clearly reduced by blending distearyl dimethyl ammonium chloride (Experimental Example 15). However, the pollen adhesion amount was not able to be reduced when other cationic surfactants that are commonly used in cosmetic products were used (Experimental Examples 16 to 19).

### <Experimental Examples 20 to 25>

Lotions having the compositions shown in Table 4 below were prepared by changing the types of oils that were blended, and the changes in the pollen adhesion amounts due to the presence or absence of distearyl dimethyl ammonium chloride were investigated in accordance with the above-mentioned evaluation method. The evaluation results are also indicated in Table 4.

**[Table 4]**

| | Exp. Ex. 20 | Exp. Ex. 21 | Exp. Ex. 22 | Exp. Ex. 23 | Exp. Ex. 24 | Exp. Ex. 25 |
|---|---|---|---|---|---|---|
| Water | 1.88 | 1.88 | 1.88 | 1.88 | 1.88 | 1.88 |
| 2-(meth)acryloyloxyethyl phosphorylcholine / (meth)acrylic acid butyl ester copolymer | 0.1 | 0.1 | 0.1 | 0.1 | 0.1 | 0.1 |
| Phenoxy ethanol | 0.02 | 0.02 | 0.02 | 0.02 | 0.02 | 0.02 |
| Mineral oil (IOB = 0.0) | 0.1 | 0.1 | - | - | - | - |
| Dimethicone (IOB = 0.19) | - | - | 0.1 | 0.1 | - | - |
| PPG-3 dipivalate (IOB = 0.52) | - | - | - | - | 0.1 | 0.1 |
| Ethanol | 97.9 | 97.89 | 97.9 | 97.89 | 97.9 | 97.89 |
| Distearyl dimethyl ammonium chloride | - | 0.01 | - | 0.01 | - | 0.01 |
| Total | 100 | 100 | 100 | 100 | 100 | 100 |
| Change in pollen adhesion amount | - | A | - | A | - | A |

As indicated in Table 4, it was observed that the pollen adhesion amount can be reduced by blending in distearyl dimethyl ammonium chloride in the case in which any one of a hydrocarbon oil, an ester oil or a silicone oil is used (Experimental Examples 21, 23 and 25).

## Claims

1. A composition for preventing adhesion of air-borne fine particles, wherein the composition contains:
(A) a 2-(meth)acryloyloxyalkyl phosphorylcholine / (meth)acrylic acid alkyl ester copolymer;
(B) a di-chain cationic surfactant represented by formula (I) below, 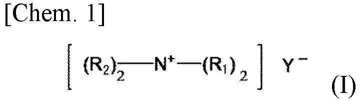 where R₁ denotes, each independently, an alkyl group having 12 to 22 carbon atoms and 0 to 3 double bonds, R₂ denotes, each independently, an alkyl group having 1 to 3 carbon atoms and lacking a double bond, and Y denotes a halogen atom, a methosulfate or a methophosphate; and
(C) one or more oils selected from among hydrocarbon oils, ester oils and silicone oils.

2. The composition for preventing adhesion of air-borne fine particles as in claim 1, wherein the (C) oil has an IOB of 0.55 or lower.

3. The composition for preventing adhesion of air-borne fine particles as in claim 1 or 2, wherein the (C) oil is of one or more types selected from the group consisting of dimethicone (IOB = 0.19), mineral oils (IOB = 0.00), cetyl ethylhexanoate (IOB = 0.13) and PPG-3 dipivalate (IOB = 0.52).

4. The composition for preventing adhesion of air-borne fine particles as in any one of claims 1 to 3, wherein the (A) 2-(meth)acryloyloxyalkyl phosphorylcholine / (meth)acrylic acid alkyl ester copolymer is a 2-(meth)acryloyloxyethyl phosphorylcholine / (meth)acrylic acid butyl ester copolymer.

5. The composition for preventing adhesion of air-borne fine particles as in any one of claims 1 to 4, wherein the (B) di-chain cationic surfactant is distearyl dimethyl ammonium chloride.

6. The composition for preventing adhesion of air-borne fine particles as in any one of claims 1 to 5, wherein the air-borne fine particles are at least one type of harmful matter that is suspended in air, selected from the group consisting of allergens such as pollen and house dust, viruses such as influenza viruses, and particulate matter such as PM2.5.
